# FASCICULE DE BREVET EUROPEEN

(11) **EP 4 103 089 B1**
(45) Date de publication et mention de la délivrance du brevet: **14.02.2024**
(21) Numéro de dépôt: 21703932.0
(22) Date de dépôt: 09.02.2021
(51) Int. Cl.: A61B 34/10, A61B 17/70, A61B 17/88, A61B 17/56

(54) **PROCÉDÉ DE CONCEPTION D'UN COUPLE DE TIGES D'UNION DESTINÉ À ÊTRE IMPLANTÉ SUR LE RACHIS D'UN PATIENT, ET PROCÉDÉ DE FABRICATION D'UNE TELLE TIGE**
VERFAHREN ZUM ENTWERFEN EINES PAARES VON AM RÜCKEN EINES PATIENTEN ZU IMPLANTIERENDEN VERBINDUNGSSTÄBEN UND VERFAHREN ZUR HERSTELLUNG EINES SOLCHEN STABES
METHOD FOR DESIGNING A PAIR OF CONNECTION RODS INTENDED TO BE IMPLANTED ON THE SPINE OF A PATIENT, AND METHOD FOR PRODUCING SUCH A ROD

(30) Priorité: 10.02.2020 FR 2001285
(43) Date de publication de la demande: 21.12.2022
(73) Titulaire: S.M.A.I.O, 69800 Saint-Priest (FR)
(72) Inventeur: ROUSSOULY, Philippe, 69004 LYON (FR); ROUSSOULY, Pierre, 69450 SAINT CYR AU MONT D'OR (FR)
(74) Mandataire: Lavoix
(86) Numéro de dépôt international: PCT/EP2021/053057
(87) Numéro de publication internationale: WO 2021/160599

(56) Documents cités:
- EP-B1- 3 057 525
- FR-A1- 3 010 628
- US-A1- 2020 015 857
- AUBERT B ET AL: "Toward Automated 3D Spine Reconstruction from Biplanar Radiographs Using CNN for Statistical Spine Model Fitting", IEEE TRANSACTIONS ON MEDICAL IMAGING, IEEE SERVICE CENTER, PISCATAWAY, NJ, US, vol. 38, no. 12, 1 décembre 2019 (2019-12-01), pages 2796-2806, XP011758292, ISSN: 0278-0062, DOI: 10.1109/TMI.2019.2914400 [extrait le 2019-11-26]

## Description

La présente invention concerne les tiges d'union, appelées aussi tiges d'ostéosynthèse rachidienne, destinées à être implantées sur la colonne vertébrale d'un patient, de manière à en corriger les déformations pathologiques par une opération d'arthrodèse rachidienne par voie postérieure. Elle concerne plus particulièrement un procédé de détermination de leur configuration tridimensionnelle optimale pour un patient donné, et la fabrication de ces tiges selon cette configuration.

Des opérations chirurgicales, appelées arthrodèses rachidiennes, visent à stabiliser un nombre plus ou moins important de vertèbres contigües, voire également à réduire une déformation de l'ensemble du rachis, c'est-à-dire à réaligner les vertèbres concernées les unes par rapport aux autres afin d'améliorer la forme statique globale de la colonne vertébrale. Des couples de tiges en métal (le plus souvent en titane ou en alliage cobalt-chrome), d'un diamètre qui est souvent de l'ordre de 3,2 mm à 7 mm, sont utilisés pour connecter entre eux des implants vertébraux. Ceux-ci sont, le plus souvent, des vis implantées sur les vertèbres considérées, auxquelles la tige est reliée via des connecteurs disposés sur chaque implant.

Deux tiges d'union sont utilisées simultanément, elles sont implantées le long du rachis, de part et d'autre des apophyses épineuses. Afin de prendre en compte les courbures rachidiennes (lordose, cyphose) de chaque patient, et de rendre possible la connexion entre la tige et les vis, on procède au cintrage de chaque tige du couple de tiges, en utilisant différentes techniques.

La plus couramment utilisée est le cintrage manuel au cours de l'opération elle-même, réalisé par le chirurgien au moyen d'une machine cintreuse manuelle comportant trois galets de cintrage. La détermination et la réalisation des rayons de courbure à imposer localement le long de la tige se fait alors selon l'estimation visuelle du chirurgien, avec la possibilité d'itérations, au cours de l'implantation de la tige, sur la valeur et l'emplacement des courbures à imposer à la tige. Le niveau de précision de cette méthode est fatalement très variable d'un opérateur à l'autre.

Des fabricants d'implants proposent des systèmes plus sophistiqués, associant l'acquisition de la position dans l'espace des têtes de vis implantées au moyen de caméras qui permettent d'obtenir les coordonnées d'une série de points correspondants, et le paramétrage d'une machine de cintrage permettant de cintrer localement la tige en fonction de la trajectoire souhaitée, qui relie l'ensemble de ces points. Le document EP-B1-2 273 944 décrit un exemple d'un tel système.

On a proposé, dans le document FR 3 010 628, de réaliser une radiographie préopératoire sagittale de la colonne vertébrale du patient à traiter, s'étendant depuis les vertèbres cervicales jusqu'aux têtes fémorales. Sur cette radiographie, on repère les paramètres pelviens, la lordose lombaire, la position de la vertèbre lombaire apicale, des distances dites SVA et SFD et un nuage de points. On déduit le morphotype auquel correspond la colonne vertébrale traitée, parmi des morphotypes prédéterminés, ainsi que le point d'apex postopératoire souhaité. On simule ensuite la correction à appliquer au segment lombaire à traiter, en définissant des arcs co-radiaux en dessous et au-dessus du point d'apex postopératoire souhaité, et en définissant deux arcs concentriques aux deux courbures obtenues, qui sont tangents entre eux au niveau du point d'apex. Après avoir translaté ces arcs à distance de la ligne moyenne de la colonne vertébrale, on peut modéliser en deux ou trois dimensions la tige à implanter, courbée selon ces arcs.

On a également proposé, dans le document EP-B1-3 057 525, de mesurer des paramètres positionnels et anatomiques sur des radiographies face et profil debout préopératoires du patient, et de donner la possibilité, via des outils de gestion d'imagerie, de couper l'image radiographique de chacune des deux incidences selon différents axes, et de réaliser des rotations entre elles des portions de l'image ainsi obtenues, afin de simuler le résultat d'une chirurgie de réalignement. Sur cette image reconstituée visant à matérialiser le résultat post-opératoire escompté, une courbe de type spline est dessinée. Ses coordonnées sont exportées vers une machine de cintrage à commande numérique qui réalise la tige sur mesure.

Il est aussi connu dans le document WO-A-2019/043426 d'ajouter à la description géométrique de la forme de dos à obtenir, un certain nombre de calculs par éléments finis censés appréhender l'ensemble des forces agissant sur la tige une fois celle-ci implantée. On tient compte, notamment, des forces musculaires et de la qualité de l'os. On détermine ainsi, en fonction du type d'alliage dont est constituée la tige et, donc, de ses propriétés mécaniques, quelle doit être sa forme au moment de sa mise en place.

Il a été démontré que fournir au chirurgien une tige cintrée numériquement dès avant l'opération d'arthrodèse rachidienne présente un triple avantage. Premièrement, cela permet un gain de temps au bloc opératoire, en évitant au chirurgien de devoir exécuter une opération qui lui prendrait quelques minutes. Deuxièmement, cela améliore la résistance mécanique de la tige, comme on évite les à-coups du cintrage manuel qui la fragilisent. Troisièmement, on matérialise de façon précise la « planification chirurgicale » à travers un cintrage respectant soigneusement la forme que le chirurgien souhaite donner à la colonne vertébrale sur le segment opéré, alors que le cintrage manuel est une source d'imprécisions pour cette forme.

L'ensemble des techniques de planification visant à obtenir un réalignement de l'ensemble bassin-rachis se fonde aujourd'hui sur des critères géométriques simples. Ceux-ci ne décrivent, cependant, que de façon très imparfaite la forme idéale du rachis pour chaque patient, en utilisant des chaines de corrélations entre un certain nombre de paramètres morphologiques et positionnels mesurés sur des radios post-opératoires de patients de référence pour lesquels les résultats cliniques ont été analysés. Les valeurs normatives retenues sont celles dont on a estimé qu'elles étaient les mieux corrélées avec la mesure d'une qualité de vie du patient satisfaisante, ces estimations se fondant sur des données relevées au moment de la prise du cliché radiographique. Le calcul de la lordose idéale, par exemple, est souvent grossier, et résume cette courbure à un angle qu'il conviendrait de calquer sur celui décrivant la forme du bassin (incidence pelvienne) à plus ou moins 10° près, sans se soucier de la répartition des angulations entre les plateaux de chaque disque et de chaque corps vertébral.

Le but de l'invention est de fournir au chirurgien un couple de tiges induisant un réalignement des vertèbres entre elles le plus proche possible d'une colonne vertébrale saine, en respectant le morphotype du patient. L'invention devrait permettre au chirurgien de déterminer la forme idéale de chaque tige du couple avant de la matérialiser au moyen d'une machine de cintrage numérique ou de toute autre technique de cintrage paramétrable. Les courbures y seraient non seulement décrites en fonction des angulations des plateaux des corps vertébraux figurant à leurs extrémités, mais également en fonction des angulations de chaque disque qui les composent.

A cet effet, l'invention a pour objet un procédé de conception d'un couple de tiges d'union destiné à être implanté sur le rachis d'un patient souffrant d'une pathologie du rachis au cours d'une arthrodèse rachidienne par voie postérieure, ce procédé de conception étant tel que défini à la revendication 1.

L'invention a également pour objet un procédé de fabrication d'une tige d'union destinée à être implantée sur le rachis d'un patient souffrant d'une pathologie du rachis au cours d'une arthrodèse rachidienne par voie postérieure, ce procédé de fabrication étant tel que défini à la revendication 6.

Des caractéristiques additionnelles avantageuses du procédé de conception et du procédé de fabrication conformes à l'invention sont spécifiées aux autres revendications.

Comme on l'aura compris, le procédé selon l'invention consiste à traiter en données d'entrée une imagerie (radiographies, scanner, IRM, projections par franges de moirée, etc.) pour laquelle on connaît précisément la taille d'un pixel, de l'ensemble bassin-rachis pathologique d'un patient, et à en déduire au moyen d'étapes que l'on détaillera ci-après la forme que doit prendre chacune des tiges qui seront implantées sur le rachis du patient pour restituer à l'ensemble bassin-rachis une morphologie saine désirée par le chirurgien. Ce traitement est typiquement couplé avec une machine de cintrage numérique ou un dispositif équivalent, susceptible de cintrer chaque tige conformément aux préconisations résultant de l'application du procédé selon l'invention.

L'invention consiste, dans un premier temps, à transformer l'imagerie en une modélisation bi- ou tridimensionnelle du rachis, dans laquelle chaque vertèbre est représentée par un tronc de cône elliptique, chaque tête fémorale par une sphère, et le plateau sacré par une ellipse. Le plus généralement, la modélisation sera tridimensionnelle. Mais si on sait par avance que la malformation de l'ensemble rachis-bassin du patient n'est présente strictement que dans le plan sagittal et que sa correction ne devrait pas entraîner de déformation supplémentaire dans le plan frontal, une modélisation bidimensionnelle peut être suffisante.

Cette modélisation permet alors de mesurer des paramètres positionnels des vertèbres entre elles et des têtes fémorales par rapport au plateau sacré, ainsi qu'un paramètre morphologique décrivant la forme du bassin, qui seront utilisés pour classer chaque patient dans un type d'alignement pathologique prédéfini. A chaque type d'alignement pathologique correspondra un type d'alignement sain fixant les paramètres morphologiques clés dans un éventail de valeurs dites normales, qui serviront de cible pour la restauration d'un équilibre satisfaisant de la colonne vertébrale et du bassin du patient.

Des outils de modification positionnelle de chaque élément de la modélisation du bassin et du rachis, simulant de façon précise les effets possibles d'une chirurgie sur l'alignement de ces derniers, afin de suggérer une configuration de réalignement, sont fournis par l'invention. L'utilisateur pourra constater, à chaque modification induite par ces outils, dans quelle mesure les paramètres positionnels des vertèbres et du bassin atteindront des valeurs dites « normales » pour la configuration cible que l'on cherche à atteindre.

Une fois que les modifications apportées à la modélisation de l'ensemble bassin-rachis (soit à la discrétion du chirurgien, soit au moyen d'un algorithme qui sera décrit plus loin), auront permis d'atteindre la configuration cible, le chirurgien pourra choisir quels segments seront fusionnés lors de la mise en place des tiges.

Les configurations de chaque tige, typiquement parallèle à la courbe de type spline passant par le centre des corps vertébraux des segments en question, sont exportées sous la forme d'un nuage de points fournissant des instructions de cintrage tridimensionnelles à une machine de cintrage, ou à tout autre dispositif de cintrage paramétrable, ce qui permet alors de réaliser un couple de tiges respectant, avec une tolérance donnée, la forme que l'on souhaite imprimer au rachis sur le segment vertébral à opérer.

L'invention sera mieux comprise au moyen de la description qui suit, donnée en référence aux figures annexées suivantes :

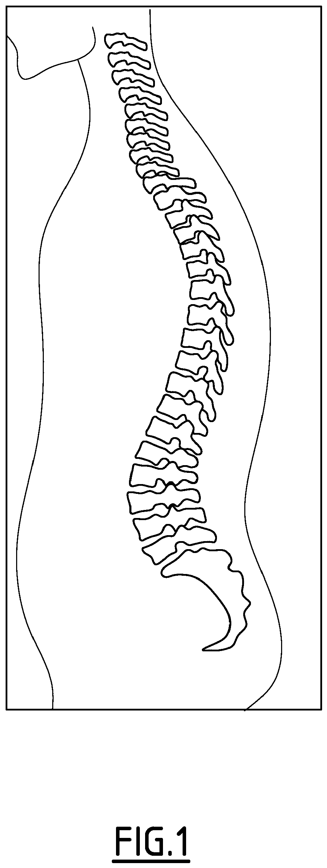

- [Fig 1] La figure 1 qui schématise dans le plan sagittal le rachis d'un patient présentant une scoliose sévère ;

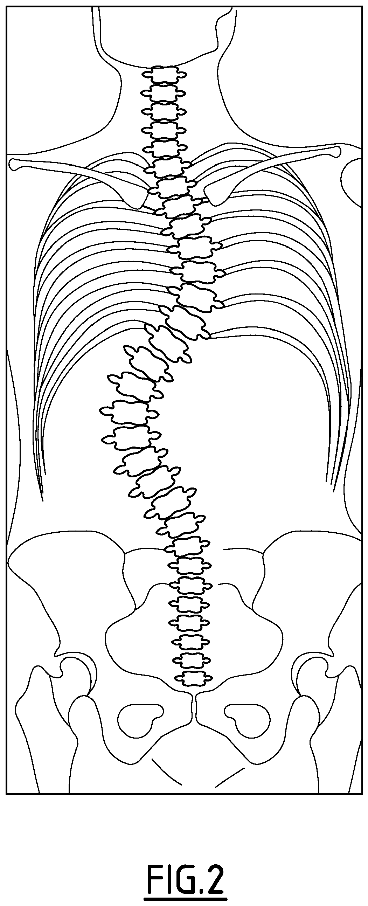

- [Fig 2] La figure 2 qui schématise dans le plan frontal le rachis de ce même patient ;

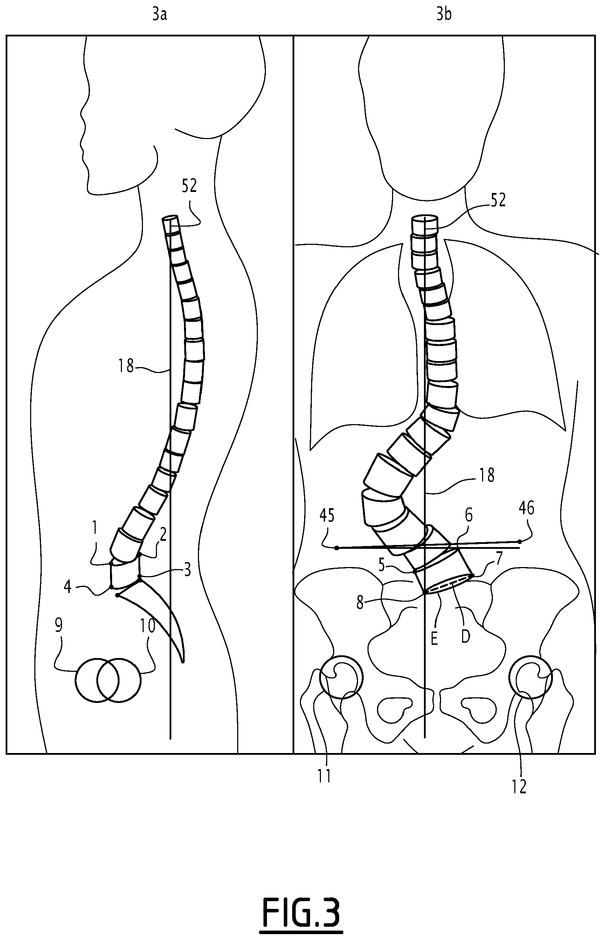

- [Fig 3] La figure 3 qui montre des portions des schématisations des figures 1 (figure 3a) et 2 (figure 3b) dans lesquelles on a matérialisé la modélisation des corps vertébraux sous forme de troncs de cônes elliptiques et de points caractéristiques et le plateau sacré sous forme d'une ellipse et de son grand axe, ainsi que la ligne verticale dite classiquement « C7 plumbline » qui passe par le centre du corps vertébral de la vertèbre cervicale C7;

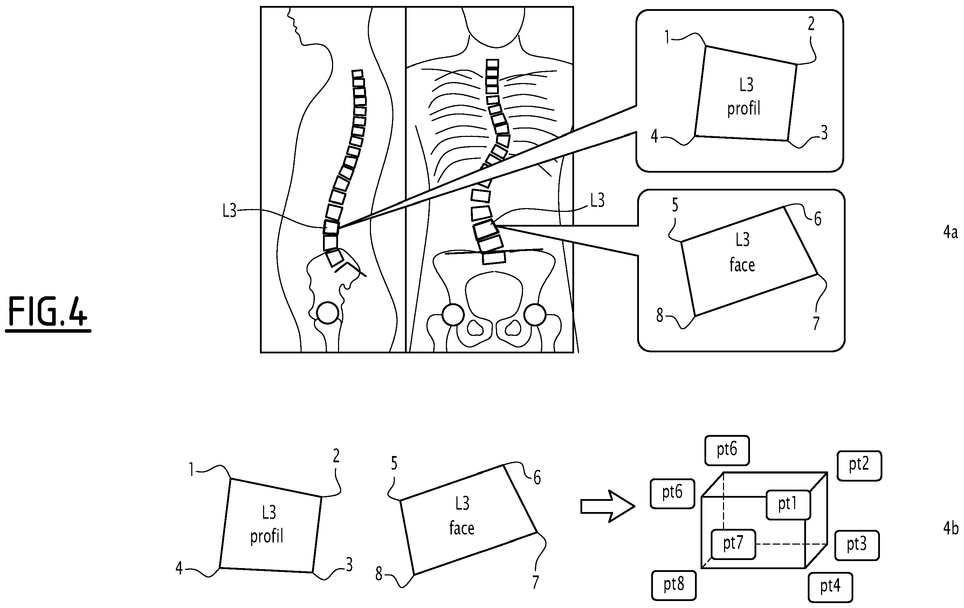

- [Fig 4] La figure 4 qui montre (figure 4a) les quadrilatères modélisant un corps vertébral donné (en l'occurrence le corps vertébral de la vertèbre L3) représenté également au sein du rachis, vu de profil et de face, et (figure 4b) la boîte englobante déduite de ces quadrilatères et la manière dont elle est déduite des quadrilatères de la figure 4a ;

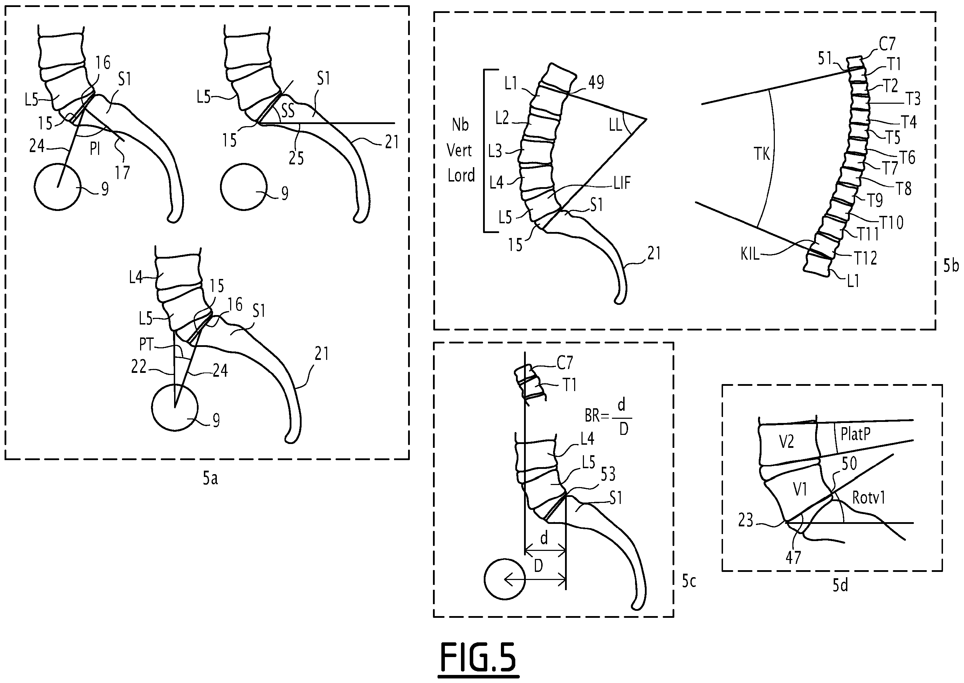

- [Fig 5] La figure 5 qui représente des portions du rachis vues dans le plan sagittal, et illustre diverses grandeurs que la modélisation prend en compte, à savoir les paramètres pelviens (figure 5a), les paramètres décrivant la forme du dos (figure 5b), les paramètres d'équilibre (figure 5c) et des paramètres locaux relatifs à deux vertèbres adjacentes (figure 5d) ;

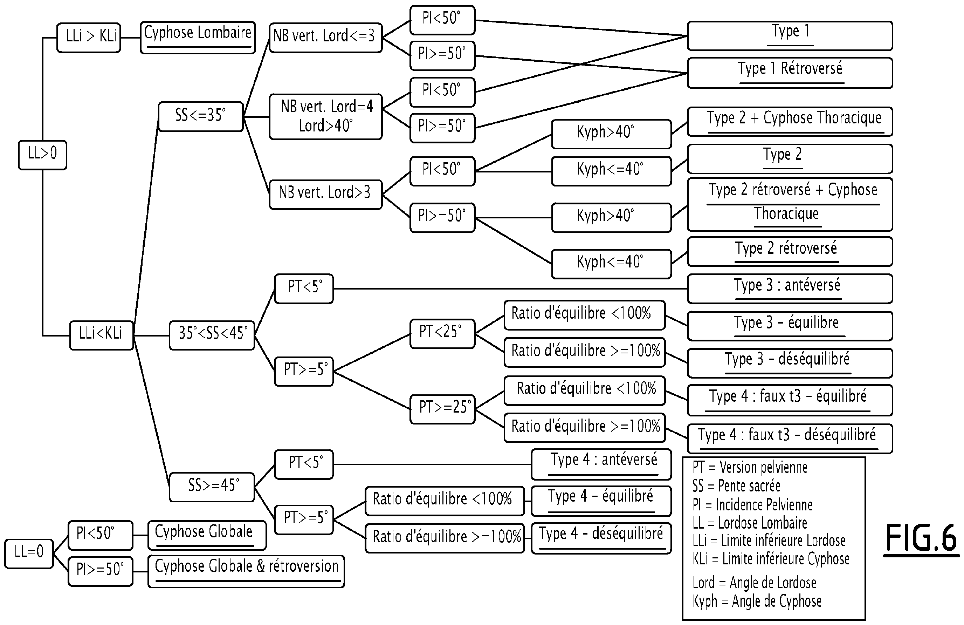

- [Fig 6] La figure 6 qui montre comment les diverses grandeurs illustrées par la figure 5 permettent de classifier un rachis sain ou pathologique ;

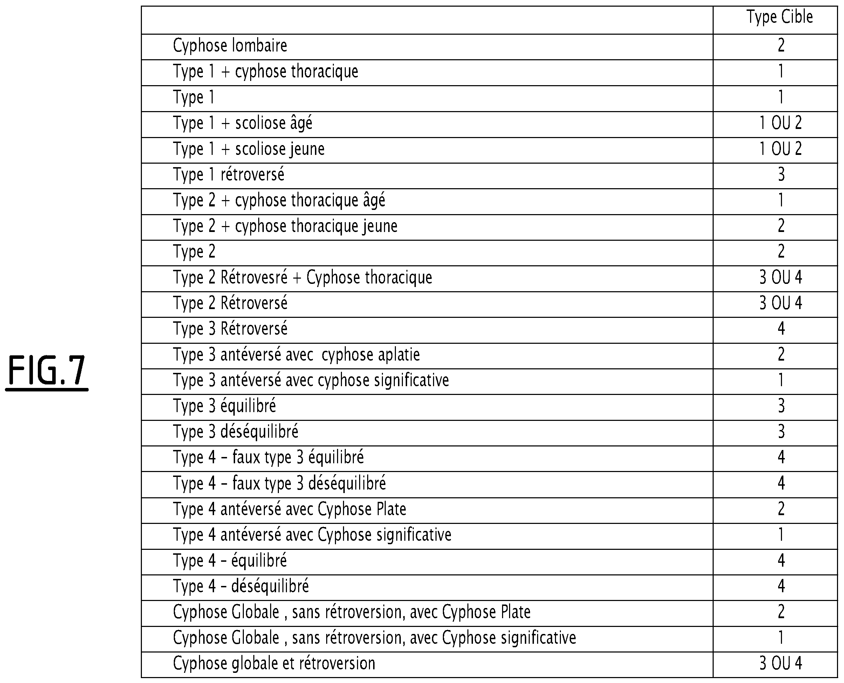

- [Fig 7] La figure 7 qui montre la correspondance entre les différents types de rachis définis à la figure 6 et le type de rachis sain selon la classification de Roussouly à quatre types dans lequel les tiges d'union conçues selon l'invention doivent ramener un rachis pathologique d'un type donné ;

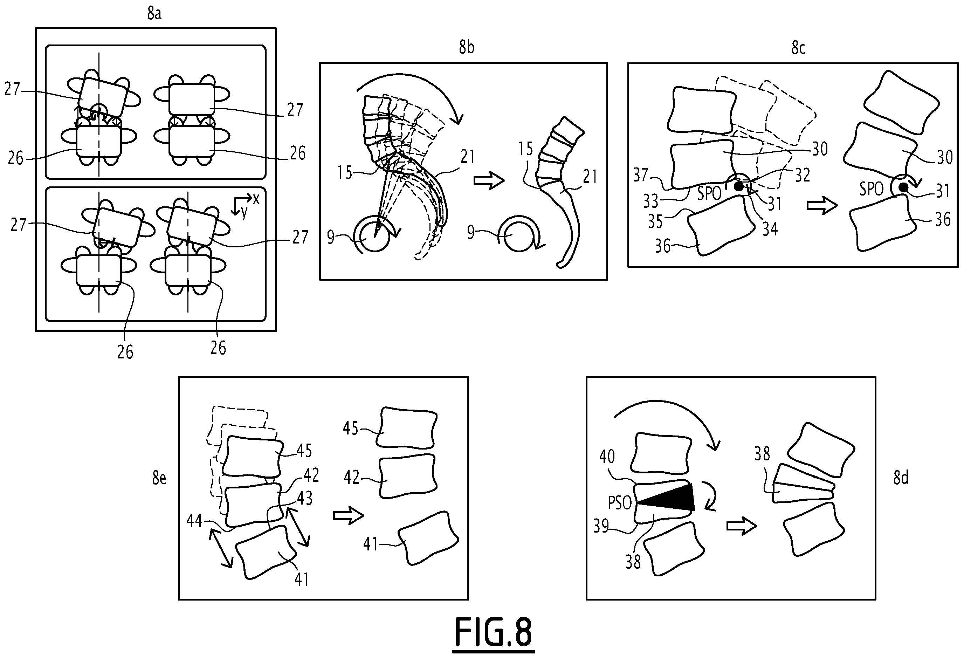

- [Fig 8] La figure 8 qui montre schématiquement le principe général de l'utilisation des cinq outils selon l'invention pour modéliser le redressement d'une portion du rachis, à savoir la figure 8a vue dans le plan frontal pour le premier outil, la figure 8b vue dans le plan sagittal pour le deuxième outil, la figure 8c vue dans le plan sagittal pour le troisième outil, la figure 8d vue dans le plan sagittal pour le quatrième outil, et la figure 8e vue dans le plan sagittal pour le cinquième outil ;

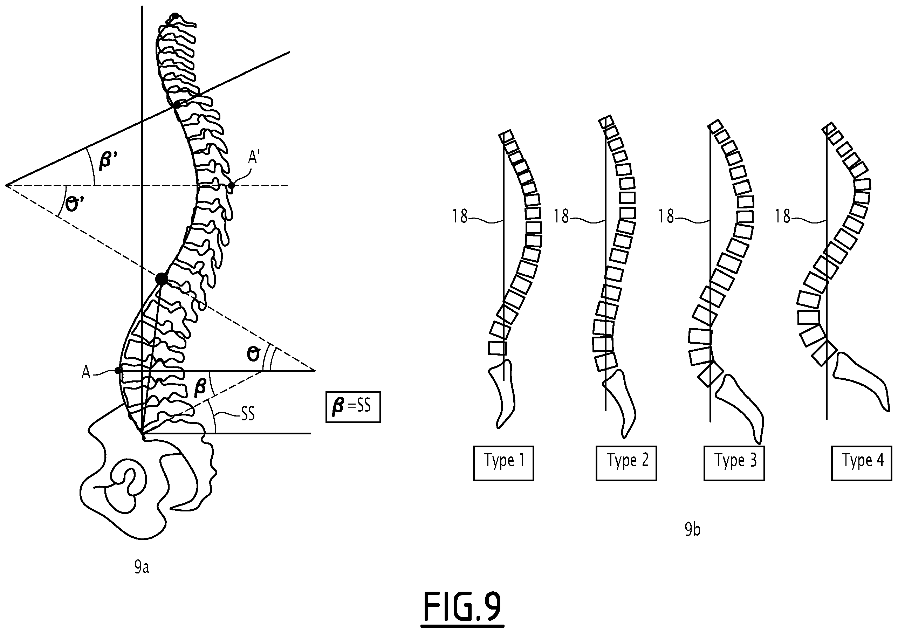

- [Fig 9] La figure 9 qui montre schématiquement dans le plan sagittal un rachis sain et les différentes grandeurs qui permettent de caractériser sa forme (figure 9a) et les différents types de rachis sains classés selon la classification de Roussouly à quatre types (figure 9b).

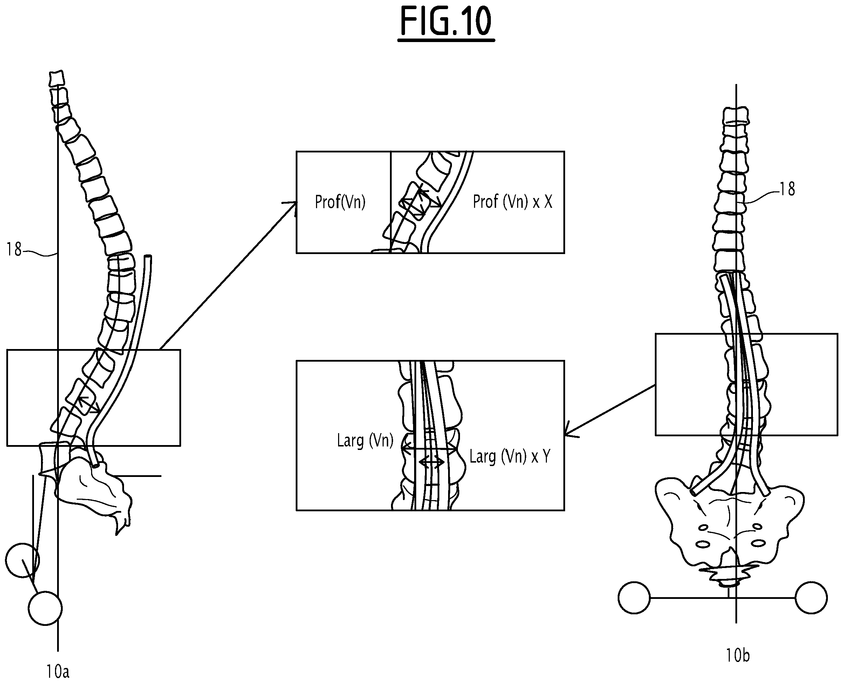

- [Fig 10] La figure 10 qui montre comment les nuages de points permettant de définir la géométrie des tiges à fabriquer se répartissent de part et d'autre de la courbure passant par le centre des corps vertébraux réalignés dans les vues sagittales comme sur la figure 10a et dans les vues frontales comme sur la figure 10b ;

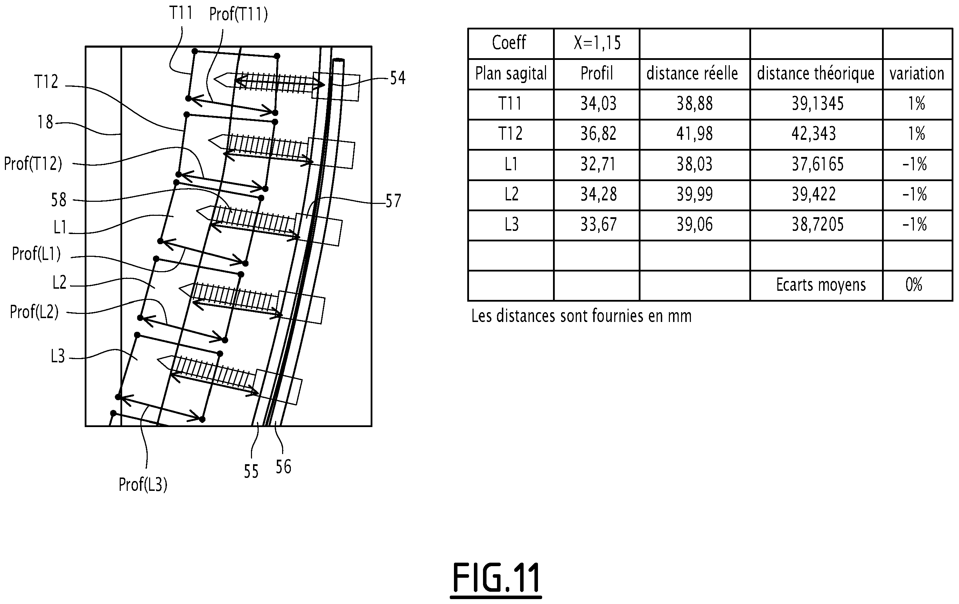

- [Fig 11] La figure 11 qui montre schématiquement dans le plan sagittal comment les vis sont typiquement implantées sur une partie du rachis, les tiges étant en place dans les connecteurs insérés sur les vis.

La première étape de l'invention consiste à réaliser des clichés du patient à traiter par un procédé d'imagerie médicale quelconque : radiographie, scanographie, IRM... La technique utilisée doit être capable de définir les contours des vertèbres avec suffisamment de netteté pour que les points qui seront à prendre en compte dans la suite du procédé selon l'invention aient leurs positions définies avec une précision convenable. Le patient est alors debout, immobile et dans une position standard. Des schémas du rachis résultant de tels clichés sont montrés sur la figure 1 pour un cliché dans le plan sagittal, et sur la figure 2 pour un cliché dans le plan frontal antérieur.

Si on sait par avance que le patient ne souffre que d'une pathologie bidimensionnelle du rachis, limitée au plan sagittal, il est suffisant de réaliser une seule série de clichés, montrant le rachis dans le plan sagittal, en vue de droite ou de gauche. En revanche, si la pathologie est tridimensionnelle, comme dans le cas d'une scoliose (c'est le cas représenté sur les figures 1 et 2), il faut réaliser deux séries de clichés, de préférence pris simultanément, ou pris sans mouvement du patient entre les deux séries. Typiquement, l'une des séries montre le rachis dans le plan sagittal en vue de droite ou de gauche (dans l'exemple décrit et représenté, toutes les figures montrant le rachis dans le plan sagittal ont été prises en vue de gauche, comme sur la figure 1), l'autre série dans le plan frontal en vue antérieure (comme sur la figure 2), en tout cas dans deux plans strictement orthogonaux. C'est à ces conditions que l'on pourra reconstituer une image fidèle du rachis et des mauvais positionnements des vertèbres, auxquels la pose des tiges d'ostéosynthèse rachidienne, que l'invention vise à conformer de façon adéquate, devra remédier.

Il faut disposer de clichés qui couvrent la zone s'étendant au moins à partir de la vertèbre cervicale C7, jusqu'aux têtes fémorales.

La tige d'ostéosynthèse ne s'étendra que sur une partie du rachis puisque dans tous les cas, comme il est usuel, elle ne sera pas reliée aux vertèbres cervicales et à la totalité du sacrum (mais elle pourra être reliée à la vertèbre S1, en particulier en cas de malposition relative des vertèbres L5 et S1).

Toutefois, une correction dans une zone pathologique du rachis a fatalement de l'influence sur des zones saines plus ou moins voisines, et disposer d'une modélisation prenant en compte l'ensemble du rachis et du bassin (hors les vertèbres cervicales supérieures qui ne seraient pas concernées par la correction et sa modélisation) permet d'apprécier le rééquilibrage global du rachis et du bassin induit par la chirurgie, ainsi que les compensations sur les segments vertébraux non fusionnés sus-jacents et, éventuellement, sous-jacents.

Il est nécessaire de prendre en compte le bassin du patient dans la modélisation, pour deux raisons.

Premièrement, la morphologie du bassin est un des éléments déterminants pour le classement du dos du patient, tel qu'on le voit sur les clichés, dans l'un ou l'autre des types connus classiquement de dos sains ou pathologiques, ce qui oblige à disposer d'une bonne visibilité sur le plateau sacré et les têtes fémorales en vue sagittale. Des critères permettant de réaliser un exemple d'un tel classement seront détaillés plus loin.

Deuxièmement, les déséquilibres de l'alignement des vertèbres sont souvent accompagnés d'une compensation au niveau du bassin (rétroversion ou antéversion), qui permet au patient de limiter le déséquilibre de sa statique générale, au prix d'efforts souvent douloureux pour lui. Concrètement, cette compensation se traduit par une rotation du bassin autour de l'axe passant par le centre des têtes fémorales en vue sagittale.

Prendre le bassin en compte permet donc de ne pas sous-estimer la quantité de correction à apporter au rachis pour le réaligner. S'il est adéquat, le réalignement s'accompagnera naturellement d'une disparition de l'effet de compensation préalablement observé au niveau du bassin, et qui ne sera plus utile au patient pour tenter de maintenir son équilibre.

Les clichés sont d'abord convertis en fichiers informatiques contenant la taille d'un pixel, typiquement selon le standard DICOM destiné à la gestion informatique des données issues de l'imagerie médicale. On peut aussi adjoindre aux clichés une échelle de distance graduée validée, permettant une calibration manuelle. Pour cela on acquiert deux points sur le cliché d'imagerie, dont on connait l'espacement réel grâce, par exemple, à un outil de calibration radio-opaque, tel qu'une bille radio-opaque, de dimension connue. On introduit ces dimensions dans le logiciel afin de connaître la dimension d'un pixel.

L'étape suivante consiste à acquérir des repères morphologiques-clés sur les clichés. Cela est fait, de préférence, au moyen d'un logiciel de modélisation dédié qui permet de poser des points sur la ou les images radiographiques. La figure 3 schématise des clichés radiographiques pris de profil dans le plan sagittal en vue de profil gauche dans l'exemple représenté (figure 3a) et de face dans le plan frontal antérieur (figue 3b) dans l'exemple représenté d'un patient présentant une scoliose sévère, donc une pathologie tridimensionnelle qui nécessite pour sa correction l'utilisation de deux clichés réalisés dans des plans orthogonaux. Concernant les vertèbres, sur chaque cliché montrant le rachis dans le plan sagittal et, le cas échéant, dans le plan frontal, on dispose des points 1, 2, 3, 4 (figure 3a), et le cas échéant 5, 6, 7, 8 (figure 3b) sur les quatre sommets de chaque corps vertébral vu en projection bidimensionnelle dans le plan de prise de vue, de façon à définir son contour par un quadrilatère dans chaque plan. Les figures 3 et 4a illustrent cette opération pour les vertèbres L5 et L3 respectivement. La même opération de localisation est réalisée sur les autres vertèbres prises en compte par la modélisation et qui sont repérées sur les figures 3a, 3b.

Les plateaux supérieur et inférieur de chaque vertèbre sont définissables chacun par une ellipse et son grand axe, souvent bien visibles radiologiquement.

Le plateau sacré est représenté, en vue sagittale sous la forme d'un segment s'il n'y a pas de déformation du rachis dans le plan frontal à son niveau, ou par un disque ellptique dans le plan sagittal s'il y a une déformation du rachis dans le plan frontal et en vue frontale, sous la forme d'un disque elliptique E, et par un segment D qui constitue le grand axe de l'ellipse E, comme on le voit sur la figure 3b. Cela permet de tenir compte de l'inclinaison du sacrum dans le plan sagittal

Puisque, comme on l'a expliqué, les deux clichés sagittal et frontal sont réalisés simultanément, ou sans changement de position du patient entre chaque cliché, le cliché sur lequel la vertèbre est la mieux visualisée permet, par projection, d'extrapoler la modélisation de la vertèbre sur l'autre cliché où elle serait moins visible, ou d'affiner cette modélisation du fait qu'on est facilement capable de connaître la latitude (c'est-à-dire la position verticale par rapport à un point de référence quelconque) de la vertèbre à reconstruire.

Les têtes fémorales sont également définies sous forme de cercles 9, 10, en vues de profil et le cas échéant, 11, 12 en vue de face, comme on le voit sur les figures 3a, 3b, 5a, 5c, 8b. Il est préférable, lors de la prise des clichés, que les cercles 9, 10 représentant les têtes fémorales dans le plan sagittal soient superposés, ou au moins très peu décalés comme c'est le cas sur la figure 3a. Un décalage trop important dans le plan antéro-postérieur est l'indice d'une position en rotation du bassin qui peut altérer les mesures à venir.

D'autres points significatifs du squelette du patient, comme les sommets 45, 46 des os iliaques du bassin (voir la figure 3b), ou le mur postérieur du sacrum et des points qui, pris ensemble, sont représentatifs de la courbure du rachis, peuvent également être acquis à cette occasion, si cela apparaît utile pour affiner la modélisation.

Les localisations des points relevés sont entrées dans le logiciel de modélisation.

Une fois les repères morphologiques acquis sur le cliché d'imagerie, des paramètres morphologiques et positionnels vont être calculés, comme représenté sur la figure 5. Ils sont classables en quatre catégories : paramètres pelviens (figure 5a), paramètres relatifs à la forme du dos (figure 5b), paramètres d'équilibre global (figure 5c) et paramètres locaux (figure 5d), ces derniers prenant en compte des couples de vertèbres voisines quelconques, alors que les trois autres catégories prennent en compte la totalité du rachis ou des portions précises de celui-ci.

Les paramètres pelviens qui sont calculés et illustrés en vue sagittale par la figure 5a sont :
- l'incidence pelvienne (en anglais « Pelvic Incidence ») Pl, à savoir l'angle entre la droite 24 reliant le centre des têtes fémorales 9 et le milieu 16 du plateau supérieur 15 de S1 et la perpendiculaire 17 au plateau 15 ;
- la pente sacrée (en anglais « Sacral Slope ») SS, à savoir l'angle formé entre le plateau supérieur 15 de S1 et l'horizontale 25 ;
- la version pelvienne (en anglais « pelvis tilt ») PT, à savoir l'angle formé entre la droite 24 reliant le centre des têtes fémorales 12 et le milieu 16 du plateau supérieur 15 de S1 et la verticale 22.

Les paramètres relatifs à la forme du dos et les paramètres locaux, qui sont calculés et illustrés en vue sagittale par les figures 5b et 5d sont les suivants.

On note Rot(Vn), pour une vertèbre Vn donnée, l'angle formé par le plateau inférieur 47 de ladite vertèbre Vn et l'horizontale comme sur l'illustration de la figure 5d pour la vertèbre V1. Par convention, le paramètre Rot(Vn), pour une vertèbre Vn donnée, est de signe positif si le bord antéro-inférieur 23 de la vertèbre Vn se trouve à une latitude inférieure à celui du bord postéro-inférieur 50 de la même vertèbre. Il est de signe négatif dans le cas contraire.

On considère, en partant du plateau sacré 15, que toute vertèbre notée Vn est incluse dans une courbure lordotique si l'une des deux conditions suivantes est remplie :
- Rot(Vn) < Rot(Vn-1) ;
- ou Rot(Vn) < Rot(Vn-2) où Vn-1 et Vn-2 sont les deux vertèbres contiguës dont la latitude se trouve immédiatement en dessous de celle de Vn (par exemple si Vn = L3, Vn-1 = L4 et Vn-2 = L5, voir la figure 5b).

D'autre part, on considère que toute vertèbre est incluse dans une courbure cyphotique si l'une des deux conditions ci-dessous est remplie : Rot(Vn) > Rot (Vn-1) ou Rot(Vn) > Rot (Vn-2).

On peut alors calculer le nombre de vertèbres incluses dans la courbure lordotique qui est notée, sur la figure 5b, « Nb vert. Lord » et qui s'étend de L1 à L5 inclusivement dans le cas représenté

On peut également définir la latitude de la vertèbre la plus basse de la première courbure lordotique notée LIF (« Lordosis Inferior Limit ») sur la figure 5b qui prend la valeur 1 si, comme représenté sur la figure 5b, il s'agit de S1, la valeur 2 pour L5, la valeur 3 pour L4, etc.

On définit aussi la latitude de la vertèbre la plus basse de la première courbure cyphotique notée KIL (« Kyphosis Inferior Limit ») sur la figure 5b, dont la valeur est calculée de la même façon. Dans le cas représenté sur la figure 5b, cette première courbure cyphotique s'étend sur toute la partie thoracique du rachis, de la vertèbre T12 à la vertèbre T1 et prend la suite de la première courbure lordotique.

On peut calculer l'angle de lordose lombaire, noté LL sur la figure 5b et défini par l'angle formé :
- Soit, comme représenté, par le plateau supérieur 15 de S1 (si S1 est bien incluse dans la courbure lordotique, comme c'est normalement le cas) et le plateau supérieur de la vertèbre de la courbure lordotique ayant la latitude la plus haute (L1 dans l'exemple représenté) ;
- Soit par le plateau inférieur de la vertèbre lombaire ayant la latitude la plus basse et le plateau supérieur 49 de la vertèbre de la courbure lordotique ayant la latitude la plus haute, en particulier si S1 n'est pas incluse dans la courbure lordotique.

On peut calculer l'angle de cyphose thoracique noté TK sur la figure 5b et défini par l'angle formé par le plateau inférieur 50 de la vertèbre de la courbure cyphotique ayant la latitude la plus basse (T12 dans l'exemple représenté), et le plateau supérieur 51 de la vertèbre de la courbure cyphotique ayant la latitude la plus haute (T1 dans l'exemple représenté).

On définit deux distances « d » et « D » qui sont représentées sur la figure 5c, et dont le rapport d/D est appelé « paramètre d'équilibre » (Balance Ratio), noté BR.

« d » est la différence d'abscisse, sur un axe horizontal H, entre le point d'intersection 52 des diagonales reliant les quatre sommets du corps vertébral de C7, vu dans le plan sagittal, et le bord postérieur 53 du plateau sacré 15.

L'axe vertical 18 auquel ledit axe horizontal H est perpendiculaire est l'axe passant par le point 52 et formé par les points situés à l'aplomb du point 52 lorsque le patient est debout. Cet axe vertical 18 correspond à ce que les orthopédistes appellent couramment la « C7 plumbline ».

« D » est la différence d'abscisse, sur ce même axe horizontal H, entre le bord postérieur 53 du plateau sacré 15 et le centre du segment joignant le centre de chacun des deux cercles 9, 10 symbolisant les têtes fémorales du patient vues dans le plan sagittal.

La grandeur « d » est positive si l'aplomb vertical de C7 est en avant de l'aplomb du bord postérieur du sacrum, négative dans le cas contraire.

La grandeur « D » est positive si l'aplomb du bord postérieur du sacrum est en arrière de l'aplomb du centre des têtes fémorales, négatif sinon.

Si d/D est de -10% à +50%, on a un ratio d'équilibre général BR correct, comme représenté sur la figure 5c. Dans le cas contraire, on a un ratio d'équilibre général BR plus ou moins pathologique. Un ratio d/D compris entre 50% et 100% est le signe d'un léger déséquilibre antérieur du patient. Un ratio d/D supérieur à 100% est représentatif d'un aplomb de C7 situé en avant des têtes fémorales, et un ratio d/D inférieur à -10% est représentatif d'un aplomb de C7 situé significativement en arrière des têtes fémorales.

Suite au calcul de chacun des paramètres évoqués ci-dessus, l'invention permet de classer les colonnes vertébrales examinées en différents types de dos qui peuvent être considérés comme « sains » ou « pathologiques ». La figure 6 illustre un exemple de telle classification. Les dos « Type 1 », « Type 2 », Type 3 équilibré », « Type 4 équilibré » sont réputés sains. Les autres types de dos sont pathologiques.

Dans cet exemple, les types de dos sains sont réputés droits dans le plan frontal et classés selon quatre catégories dite « types de Roussouly » qui résultent de l'analyse d'une population de plus de 646 individus ne souffrant pas de pathologies rachidiennes et pour lesquels les paramètres sus-mentionnés ont été mesurés et analysés statistiquement dans le plan sagittal (voir l'article « Description of the Sagittal Alignment of the Degenerative Human Spine » (A. Sebaaly, P. Grobost, L. Mallam, P. Roussouly), European Spine Journal (2018) 27 : 489-496),

Ainsi, les patients sont-ils classés en fonction :
- De la forme de leur bassin en prenant en compte l'incidence pelvienne IP ;
- Du positionnement de leur bassin en prenant en compte la pente sacrée SS ou la version pelvienne PT pour déceler l'existence de compensations ;
- De la forme de la colonne vertébrale en prenant en compte :
   ∘ La longueur en nombre de vertèbres de la courbure lordotique, et son positionnement sur la colonne (par exemple, sur certains cas très pathologiques, la première courbure peut être cyphotique ; et dans les cas extrêmes, la colonne vertébrale entière peut être en cyphose) ;
   ∘ Les valeurs angulaires maximales de chacune des courbures lordotiques et cyphotiques calculées dynamiquement comme décrit plus haut.
- De l'équilibre global du patient en utilisant les valeurs du « Balance ratio »

Si le patient a été classé dans l'un des quatre types de dos sains, il n'y a, par définition, pas matière à effectuer un réalignement des vertèbres. Si au contraire, il appartient à l'un des types de dos pathologiques cités par la figure 6, on déduit le type de dos « sain » auquel devrait correspondre le patient en utilisant la table de correspondance constituant la figure 7.

La classification des dos sains, dont le principe et les modalités sont donc connus en eux-mêmes, sera mieux comprise à l'aide de la figure 9.

La figure 9a représente un rachis et un bassin de configurations saines et courantes du type 3 tel qu'il sera défini plus loin (en y incluant les vertèbres cervicales dont on a vu que, à part C7, elles n'étaient pas forcément intégrées à la modélisation mise en oeuvre par l'invention), et on y a repéré des points et des grandeurs représentatifs, qui contribuent au classement du dos du patient dans l'une des quatre catégories précitées de dos sains et qui seront détaillées plus loin.

On retrouve sur la figure 9a :
- la pente sacrée SS telle qu'on l'a définie précédemment ;
- le point A (que l'on qualifie d'apex), qui est le point le plus antérieur de la face antérieure de la partie lordotique du rachis ;
- l'angle β dit « arc inférieur de la lordose » qui est l'angle formé par le plateau sacré 15 et l'horizontale passant par A ;
- le point d'inflexion IP qui est le point antéro-supérieur du plateau vertébral d'une vertèbre (T11 dans le cas représenté) où le rachis passe d'une configuration lordotique à une configuration cyphotique ;
- le point A' (qui est un autre apex), qui est le point le plus postérieur de la face antérieure de la partie cyphotique du rachis ;
- l'angle θ dit « arc supérieur de la lordose » qui est l'angle formé par l'horizontale passant par A et le plateau vertébral de la vertèbre auquel appartient le point d'inflexion IP ;
- l'angle θ' dit « arc inférieur de la cyphose », qui est l'angle formé par l'horizontale passant par A' et le plateau vertébral de la vertèbre auquel appartient le point d'inflexion IP ;
- l'angle β' dit « arc supérieur de la cyphose », qui est l'angle formé par l'horizontale passant par A' et le plateau vertébral supérieur de la vertèbre T1.

Selon cette classification, les organisations spino-pelviennes des sujets sains se subdivisent en quatre types de dos, illustrés sur la figure 9b.
- Le type 1 comprend de deux à quatre vertèbres (trois dans l'exemple représenté) dans la lordose, qui n'a qu'un seul rayon de courbure et est surmontée d'une cyphose thoraco-lombaire plus ou moins prononcée ; la pente sacrée SS est faible, inférieure ou égale à 35° ;
- Le type 2 correspond à des pentes sacrées SS faibles (inférieures ou égales à 35°). Comme on le voir sur la figure 9b, les courbures rachidiennes sont peu prononcées ; la lordose s'étend sur quatre vertèbres ou plus (six dans l'exemple représenté) ;
- Le type 3 correspond à des pentes sacrées SS moyennes (supérieures à 35° et inférieures à 45°), avec des courbures rachidiennes prononcées et des lordoses de quatre vertèbres ou plus (six dans l'exemple représenté) ;.
- Le type 4 correspond à de grandes pentes sacrées (supérieures ou égales à 45°) avec des courbures rachidiennes très prononcées et une lordose de quatre vertèbres ou plus (sept dans l'exemple représenté).

Comme on le voit sur le schéma général d'organisation spino-pelvienne de la figure 9, pour les types 2 à 4 on décompose chaque courbure rachidienne (la lordose qui ramène le rachis en arrière, et la cyphose qui le projette en avant) en deux sous-courbures (arc inférieur/arc supérieur) ayant des rayons de longueurs différentes. Ces sous-courbures sont délimitées par les apex vus précédemment (A pour la lordose et A' pour la cyphose, voir figure 9a) qui sont des points de jonction pour lesquels la tangente à la courbe passant par le centre des corps vertébraux est parfaitement verticale. On voit géométriquement que l'arc inférieur β de la lordose est égal à la pente sacrée SS. On a observé par ailleurs que l'arc supérieur θ de la lordose est constant, et proche de 20° quel que soit le type de dos. L'arc inférieur θ' de la cyphose mesure lui aussi autour de 20° alors que l'arc supérieur β' de la cyphose est lui aussi constant, avoisinant les 30°.

Pour les types 1, la lordose n'a qu'un seul rayon de courbure, généralement très aigu. La cyphose est dite thoraco-lombaire car elle s'étend non seulement dans la région thoracique, mais aussi dans la région lombaire (elle inclut L2 et L1, parfois aussi L3).

L'invention permet donc de transformer les points caractérisant la modélisation du rachis telle qu'elle a été réalisée à partir de l'imagerie du patient en l'une des quatre configurations saines qu'on vise à atteindre au moyen de la chirurgie. Dans cette optique, cinq outils sont proposés à l'utilisateur afin qu'il les utilise successivement pour obtenir la modélisation du rachis à l'état corrigé visé par le chirurgien. Il disposera ainsi d'une liste d'actions correctives à mettre en oeuvre durant la chirurgie pour parvenir à cet état d'équilibre à partir de la modélisation du rachis pathologique que l'on a décrite, comme on le verra sur les figures suivantes, et pourra en déduire la forme qu'il faut conférer aux tiges d'union pour obtenir cet état d'équilibre.

Les buts poursuivis sont les suivants :
- Le réglage de l'angulation entre les plateaux des disques intervertébraux sur le segment du rachis opéré ;
- Le réglage de l'angulation entre les plateaux inférieurs et supérieurs de chaque corps vertébral sur le segment du rachis opéré ;
- Le réglage de la hauteur des disques sur le segment du rachis opéré ;
- Le bon positionnement relatif escompté du plateau sacré et du centre des têtes fémorales suite à la chirurgie ;
- La modification post-chirurgicale attendue de l'angulation des disques intervertébraux sous-jacents et sus-jacents au segment du rachis opéré.

Un premier outil, dont l'effet est illustré par la figure 8a, permet de réaligner les vertèbres 26, 27 dans le plan frontal selon un pourcentage de réalignement allant de 0% (pas de modification) à 100% (parallélisation et alignement vertical parfaits des plateaux inférieurs de chaque corps vertébral vus dans le plan frontal). On calcule, pour chaque disque séparant deux vertèbres adjacentes Vn (la vertèbre supérieure) 27 et Vn-1 (la vertèbre inférieure 26), en vue frontale l'angle formé par le plateau inférieur de la vertèbre Vn et l'horizontale d'une part, et la différence d'abscisse entre le centre du plateau inférieur de la vertèbre Vn et le centre du plateau inférieur de la vertèbre Vn-1. Il est alors possible de ramener cet angle et cette distance à 0 si on fixe le paramètre de correction à 100%, ce qui produit un réalignement parfait des vertèbres Vn et Vn-1 sur le plan frontal, ou de laisser une partie de cette angulation et de cette distance proportionnelle au pourcentage de réalignement fixé. Par exemple, si on fixe ce pourcentage à 50%, l'angulation entre le plateau inférieur de chaque vertèbre et l'horizontale sera réduite de moitié, par rotation de la vertèbre autour du centre de son plateau inférieur. La différence d'abscisse entre le milieu du plateau inférieur de chaque vertèbre et celui de la vertèbre contiguë ayant une latitude inférieure sera également réduite de moitié par translation selon un axe horizontal.

Un deuxième outil, illustré par la figure 8b, permet de réaliser une rotation de la modélisation du plateau sacré 15 dans le plan sagittal par rapport au centre du segment joignant le centre des deux sphères 9 symbolisant les têtes fémorales, en jouant sur la correction de la version PT (appelée aussi « pente pelvienne »), afin de simuler l'annulation de la compensation pelvienne consécutive à la correction des courbures rachidiennes. Cet outil permet géométriquement de réaliser une rotation de tous les points symbolisant le plateau sacré 15 et les corps vertébraux, avec pour centre de rotation le centre des têtes fémorales 9 du patient.

Un troisième outil, illustré par la figure 8c, permet de modifier l'angulation entre les plateaux adjacents de chaque disque intervertébral (disques non représentés ; ils remplissent les espaces séparant les corps vertébraux) dans le plan sagittal en effectuant une rotation sur chaque vertèbre de latitude supérieure à celle du disque pour simuler l'effet d'une ostéotomie transforaminale de type « Smith Petersen », et/ou l'effet lordosant d'une arthrodèse alignant les corps vertébraux 30, 36 sur la tige d'union préalablement cintrée. Le centre de rotation est le milieu du segment 31 joignant le sommet postéro-inférieur 32 du plateau du corps vertébral de la vertèbre sus-jacente 30 et le sommet postéro-supérieur 34 du plateau du corps vertébral de la vertèbre sous-jacente 36. La rotation concerne tous les points sus-jacents au disque intervertébral considéré, y compris les deux points 32, 37 correspondant aux extrémités du plateau inférieur 33 du corps vertébral de la vertèbre sus-jacente 30. Cette rotation est appelée correction « SPO »

Un quatrième outil, illustré par la figure 8d, permet de modifier l'angulation entre les plateaux inférieurs et supérieurs de chaque vertèbre 38 dans le plan sagittal, en effectuant une rotation dont le centre est le centre du segment joignant le sommet antéro-inférieur 39 et le sommet antéro-supérieur 40 du corps vertébral de la vertèbre 38 considérée. La rotation concerne tous les points dont l'ordonnée est supérieure à celle du centre de rotation, et est appelée correction « PSO » car elle nécessitera pour sa réalisation une soustraction d'une portion triangulaire du corps vertébral analogue à l'opération appelée « ostéotomie de soustraction pédiculaire » (« Pedicle Substraction Osteotomy ») classiquement réalisée en chirurgie orthopédique.

Un cinquième outil, illustré par la figure 8e, permet de modifier la hauteur d'un disque (non représenté), définie comme la longueur du segment perpendiculaire au plateau supérieur 43 du corps vertébral de la vertèbre sous-jacente 41, joignant le milieu de ce dernier au point d'intersection avec le plateau inférieur 44 de la vertèbre sus-jacente 42, en effectuant une translation de tous les points sus-jacents au disque, y compris les points formant le plateau inférieur de la vertèbre sus-jacente, sur une distance définie par l'utilisateur. Cette correction est appelée correction « disk height » ou « correction cage ». Elle correspond à l'effet qu'aurait l'insertion d'une cage intersomatique dans le disque d'un patient.

La présente invention permet de transformer la modélisation tridimensionnelle en troncs de cônes elliptiques (voir la figure 3) d'un rachis en utilisant successivement chacun des cinq outils précités, soit à la discrétion de l'utilisateur, soit selon tout algorithme permettant d'atteindre les objectifs de correction pour chacun des types de dos définis par la figure 7.

Par exemple, dans une première étape, quel que soit le type de dos pathologique, on utilise l'outil 1 en fixant la correction dans le plan frontal à 100% sur l'ensemble des vertèbres de la colonne vertébrale. Les milieux des plateaux inférieurs de chaque corps vertébral vont être alignés verticalement, et les plateaux inférieurs de chacun des corps vertébraux vont prendre une position parfaitement horizontale, selon les modalités décrites plus haut au sujet de l'outil 1 décrit par la figure 8a.

Dans une deuxième étape, dans le plan sagittal, on utilise le deuxième outil pour corriger la version pelvienne PT afin qu'elle soit comprise entre 10% et 1/3 de l'incidence pelvienne Pl, restaurant ainsi un positionnement pelvien théorique conforme à celui d'un individu sain. Son action est visible dans le plan sagittal sur la figure 8.b. Au moyen de ce deuxième outil, on effectue une rotation de l'image du sacrum 21, en prenant pour centre de rotation le milieu du segment qui joint les centres des deux cercles 9, 10 symbolisant les têtes fémorales. On impose ainsi la valeur désirée à la version pelvienne PT, en restaurant un positionnement pelvien théorique conforme à celui d'un individu sain qui n'aurait pas besoin de compensation pelvienne.

Dans une troisième étape, si le patient correspond à un type de dos dont l'objectif de restauration est un type 2, 3 ou 4,
- On utilise le troisième outil sur les disques L5-S1, L4-L5 et L3-L4 en réalisant des corrections de valeur identiques dans chaque disque (sur un profil gauche, rotation côté horaire) jusqu'à ce que rot(L3) soit de signe négatif ;
- On utilise le troisième outil sur les disques L2-L3, L1-L2 et T12-L1 afin d'obtenir une angulation en lordose entre le plateau inférieur de L3 et le plateau inférieur de T12 égale à 20° (± 2°) ;
- On utilise le troisième outil sur les disques T11-T12, T10-T11, T9-T10, T8-T9, T7-T8, T6-T7, T5-T6 afin d'obtenir une angulation en cyphose entre le plateau inférieur de T12 et le plateau supérieur de T5 égale à 20° (± 2°) ;
- On utilise le troisième outil sur les disques T4-T5, T3-T4, T2-T3, T1-T2, C7-T1 afin d'obtenir une angulation en cyphose entre le plateau inférieur de T4 et le plateau supérieur de C7 égale à 30° (± 2°) ;
- Si le ratio d'équilibre atteint alors des valeurs normatives citées plus haut, on stoppe le processus. Dans le cas contraire, on utilise le quatrième outil sur L4 ou L5 jusqu'à ce que le ratio d'équilibre global GR = d/D atteigne des valeurs dites normales (-10% à 100%).

Si le patient correspond à un type de dos dont l'objectif de restauration est un type 1 :
- On n'utilise le troisième outil que pour les disques compris entre L5-S1 et L3-L4, Là encore, on cherche à atteindre un ratio d'équilibre global GR de -10 à +50% ;
- On utilise le troisième outil sur tous les disques de la partie basse de la cyphose thoraco-lombaire L2-L3, L1-L2, T12-L1, T11-T12, T10-T11 en prenant bien soin de conserver pour chaque vertèbre n, rot(Vn) > rot(L3) ;
- Si le ratio d'équilibre global GR atteint alors des valeurs normatives citées plus haut, on stoppe le processus ; dans le cas contraire, on utilise le quatrième outil sur L4 ou L5 jusqu'à ce que le ratio d'équilibre global GR atteigne des valeurs dites normales (-10% à 100%).

Une fois que la modélisation en troncs de cônes elliptiques des vertèbres et de leur alignement a été modifiée de façon à reproduire un alignement vertébral jugé convenable par le praticien conformément à la table de correspondances de la figure 7, c'est-à-dire, en général, conforme aux configurations observées dans les populations non pathologiques (position du bassin, angulation entre les corps vertébraux, aplomb de C7 par rapport au sacrum et aux têtes fémorales) en fonction du type de dos du patient, un nuage de points est calculé pour décrire une courbe passant par le centre de chaque corps vertébral sur les segments qui ont été modifiés par la simulation.

En suivant l'alignement des pédicules vertébraux et en se positionnant au niveau des arcs postérieurs des vertèbres, on peut définir une courbe caractérisant la position de réduction souhaitée du rachis déformé après correction virtuelle, dont on peut extraire la forme qu'il faut conférer à chacune des deux tiges d'union, en construisant deux autres nuages de points, situés chacun de part et d'autre du premier, et qui définissent les formes que prendront chacune des tiges après leur cintrage avant d'être implantées sur le rachis du patient.

Quand la correction est idéale, un parallélisme des deux tiges est réalisable, sauf dans la partie la plus inférieure du rachis lombaire, lorsque les tiges doivent être fixées sur les os iliaques du patient. Si c'est le cas, les points de fixation des tiges sur chacun des os iliaques sont sensiblement plus écartés l'un de l'autre que ne le sont les points de fixation des tiges sur une même vertèbre. Il est alors préférable de commencer à augmenter l'écartement des deux tiges au niveau des dernières vertèbres lombaires, de façon à ne pas devoir déformer les tiges trop brutalement, avec les risques de fragilisation des tiges qu'une variation brutale de leurs rayons de courbure procurerait.

Toutefois, en chirurgie de la déformation et en particulier chez l'adulte, la persistance d'un certain degré de déformation dans le plan frontal (scoliose résiduelle) est parfois nécessaire. Dans ce cas le parallélisme des tiges n'est plus possible, et chaque tige doit être évaluée en fonction de la combinaison des courbures résiduelles frontales et des corrections sagittales, qui imposent des rayons de courbure localement différents pour chacune des tiges, au point de rendre les deux tiges du dispositif d'arthrodèse rachidienne franchement non parallèles, au moins dans certaines portions du rachis.

Les deux nuages de points qui sont déduits du nuage de points initial, c'est-à-dire celui passant par les centres des corps vertébraux, permettent de déduire la forme précise qu'il faut conférer à chacune des tiges. Ils passent par une série de points théoriques situés de part et d'autres de la courbe passant par le centre des corps vertébraux dans le plan frontal, et postérieurement à cette dernière dans le plan sagittal afin de correspondre à l'emplacement du connecteur de chaque vis pédiculaire implantée. Il faut donc, le cas échéant, prévoir un décalage entre le lieu effectif d'implantation de la vis sur la vertèbre et le point correspondant du nuage de points déduit du nuage initial et par lequel doit passer la tige. Ce décalage est à paramétrer en fonction du type d'instrumentation, notamment du type de connecteur utilisé à cet endroit précis du rachis pour relier la tige à la vis et de la distance qu'il impose entre l'axe de la tige et l'axe de la vis.

Ainsi, la figure 10 montre comment les nuages de points permettant de définir la géométrie des tiges à fabriquer se répartissent de part et d'autre de la courbe théorique 54 passant par le centre des corps vertébraux réalignés dans les vues sagittales comme sur la figure 10a et dans les vues frontales comme sur la figure 10b. Deux paramètres X et Y qui sont fonction de la profondeur et de la largeur du corps vertébral de chaque vertèbre concernée par l'arthrodèse doivent être fixés. Ils dépendent du type d'instrumentation utilisée et permettent de définir le positionnement des courbures de chaque tige respectivement postérieurement à la courbe sagittale et de part et d'autre de la courbe frontale passant par le centre des corps vertébraux.

Ainsi, comme on le voit par exemple sur la figure 11 pour laquelle on a choisi des dispositifs pourvus chacun d'un connecteur 57 à chargement latéral de type dit « low profile » inséré sur une tige (« post ») prolongeant la partie filetée 58 de la vis, tout d'abord dans le plan sagittal pour un patient réel sur le segment instrumenté de T11 à L3, pour chaque corps vertébral, on peut calculer, pour chaque corps vertébral T11, T12, L1, L2, L3 un rapport de distance entre :
- la profondeur Prof(T11), ProfT(12), Prof(L1), Prof(L2), Prof(L3) dudit corps vertébral, vu dans le plan sagittal et matérialisé par le quadrilatère défini précédemment pendant la modélisation (cf figure 4) ;
- et la distance entre le centre du corps vertébral T11, T12, L1, L2, L3 (le point d'intersection des diagonales du quadrilatère représentant ledit corps vertébral) et l'axe de la tige 55 implantée postérieurement sur ce dernier et positionnée dans le connecteur 57 correspondant à la vis implantée dans ledit corps vertébral.

Un exemple de tableau de mesures correspondant est annexé à la figure 11. Sur le cas considéré, en positionnant la courbe 54 décrivant la tige théorique postérieurement à la courbe passant par le centre des corps vertébraux réalignés après planification avec un paramètre X = 1,15 fois la longueur du plateau inférieur du corps vertébral pour chaque niveau, on obtient une tige très proche de celle que le chirurgien a réellement implantée : les écarts entre la distance réelle entre l'axe de la tige 55 et le centre du corps vertébral sur lequel la vis correspondante est implantée sont de l'ordre de ± 1%. Il est donc possible d'atteindre une grande précision dans la conformation et l'installation des tiges 55, 56 et des ensembles vis-connecteurs 57, 58.

La même logique peut être appliquée pour la courbure dans le plan frontal, en appliquant cette fois-ci un paramètre Y, de part et d'autre de la courbe 54 passant par le centre des corps vertébraux T11, T12, L1, L2, L3.

Le chirurgien réalise le cintrage de chacune des tiges selon les indications que le procédé de conception des tiges selon l'invention lui a fournies. Ce cintrage peut être réalisé à l'aide d'une machine cintreuse que le chirurgien utilise selon les indications que la modélisation précédemment décrite lui a fournies. Mais il est préférable, pour des raisons évidentes de facilité, de rapidité d'exécution et de précision du cintrage que cette opération soit réalisée au moyen de machines à commande numérique connues en elles-mêmes. A cet effet, la géométrie souhaitée pour une tige donnée est convertie en données informatiques, de façon classique, et ces données sont transmises à la machine de cintrage.

On peut, si besoin, ajouter des points supplémentaires à chacun des nuages utilisés pour la mise en forme d'une tige. Ces points supplémentaires ne correspondent pas forcément à des points particuliers du rachis dans sa position corrigée, mais servent à donner des indications au chirurgien, ou à paramétrer la machine de cintrage, de manière à rendre plus progressifs les changements de courbure de la tige entre deux points d'ancrage. Des efforts moindres sont ainsi appliqués à la tige lors de sa conformation, limitant ainsi les risques de fissuration, voire de rupture, de la tige lors de sa conformation, puis postérieurement à son implantation sur le rachis du patient.

## Revendications

1. Procédé de conception d'un couple de tiges d'union (55, 56) destiné à être implanté sur le rachis d'un patient souffrant d'une pathologie du rachis au cours d'une arthrodèse rachidienne par voie postérieure, **caractérisé en ce que** :
- on construit une modélisation tridimensionnelle du rachis pathologique, en procédant de la façon suivante :
* on réalise au moins un cliché du rachis du patient à traiter, au moyen d'un procédé d'imagerie médicale, à savoir un cliché montrant le rachis dans le plan sagittal, et, si ladite pathologie est tridimensionnelle, un autre cliché montrant le rachis dans le plan frontal en vue antérieure, le patient étant dans la même position que pour la réalisation du cliché dans le plan sagittal, lesdits clichés couvrant au moins la zone du rachis s'étendant de la vertèbre cervicale C7 jusqu'à l'extrémité du sacrum et incluant les têtes fémorales du patient ;
* à partir du ou desdits clichés, on acquiert des repères morphologiques en disposant des points sur les quatre sommets de chaque corps vertébral vu en projection bidimensionnelle dans le plan de prise de vue de chaque cliché, de façon à définir les contours desdits corps vertébraux sur chaque cliché par un quadrilatère ;
* on construit, à partir du ou desdits clichés, une modélisation tridimensionnelle simplifiée de chaque corps vertébral sous forme d'un tronc de cône elliptique pour lequel chaque corps vertébral est représenté dans le plan dudit cliché, ou dans les plans respectifs desdits clichés, par un quadrilatère défini par quatre points (1, 2, 3, 4 ; 5, 6, 7, 8) placés sur les quatre sommets de chaque corps vertébral vu en projection bidimensionnelle dans le ou lesdits plan(s), où chaque plateau vertébral est représenté par une ellipse ;
* on définit le plateau sacré (15) par un segment dans le plan sagittal, s'il n'y a pas de déformation du rachis dans le plan frontal, ou par un disque elliptique dans le plan sagittal s'il y a une déformation du rachis dans le plan frontal à son niveau, et par un disque elliptique dans le plan frontal dans tous les cas pour tenir compte de l'inclinaison du sacrum dans le plan sagittal ;
* on définit par des cercles (9, 10) les projections des contours des têtes fémorales dans les plans des prises de vue des clichés ;
* on définit le segment qui relie le centre des cercles (9, 10) représentant les têtes fémorales si leurs projections dans le plan sagittal sont décalées, ou le point représentant le centre desdits cercles s'ils sont confondus dans le plan sagittal ;
* on mesure sur les clichés l'incidence pelvienne (PI), la pente sacrée (SS) et la version pelvienne (PT) ;
* on définit sur les clichés la « C7 plumbline » (18) du rachis que l'on prend comme axe vertical de référence et qui passe par un point situé au milieu du corps vertébral de C7;
* on définit le ratio d'équilibre général (d/D), où d est la distance entre l'axe vertical passant par le milieu de la vertèbre C7 et l'axe vertical passant par l'extrémité postérieure du segment représentant le plateau sacré (15) dans le plan sagittal, et D est la distance entre un axe vertical passant par ladite extrémité postérieure du segment représentant le plateau sacré dans le plan sagittal et le milieu du segment qui relie les centres des cercles représentant les têtes fémorales ou le point représentant le centre desdits cercles s'ils sont confondus dans le plan sagittal ;
* on définit sur les clichés les limites des courbures lordotiques, et cyphotiques, et on calcule le nombre de vertèbres inclues dans la lordose lombaire.
- on utilise ladite modélisation tridimensionnelle pour classer le rachis pathologique du patient dans une catégorie donnée d'une classification préétablie des types de dos pathologiques ou sains, et on en déduit une future position corrigée des vertèbres du rachis que l'on vise à obtenir, si besoin, ladite future position corrigée relevant d'une catégorie préétablie des types de dos sains ;
- on modélise le rachis du patient dans sa future position corrigée, en prenant en compte les configurations et les positions des corps vertébraux du rachis, du plateau sacré (15) et des têtes fémorales, en utilisant successivement cinq outils en partant de ladite modélisation :
* à l'aide d'un premier outil, on réaligne les vertèbres dans le plan frontal, selon un pourcentage de réalignement allant, pour chaque vertèbre, de 0%, ce qui correspond à une absence de réalignement, à 100%, ce qui correspond à une parallélisation parfaite des plateaux inférieurs de chaque corps vertébral dans le plan frontal et à un alignement vertical des milieux desdits plateaux inférieurs ;
* à l'aide d'un deuxième outil, on réalise une rotation de la modélisation du plateau sacré dans le plan sagittal par rapport au centre du segment joignant les centres des deux sphères symbolisant les têtes fémorales en jouant sur la correction de la version (PT) ;
* à l'aide d'un troisième outil, on modifie l'angulation dans le plan sagittal entre les plateaux adjacents de chaque disque intervertébral que l'on souhaite opérer, en effectuant une rotation sur chaque vertèbre, le centre de rotation étant le centre du segment joignant le bord postéro-inférieur du plateau du corps vertébral de la vertèbre sus-jacente et le bord postéro-supérieur du plateau du corps vertébral de la vertèbre sous-jacente, et la rotation concernant tous les points sus-jacents au disque intervertébral considéré, y compris les deux points du plateau inférieur du corps vertébral de la vertèbre sus-jacente ;
* à l'aide d'un quatrième outil, on modifie l'angulation entre les plateaux inférieurs et supérieurs d'une à deux vertèbres dans le plan sagittal, comme on le ferait lors d'une correction d'ostéotomie transpédiculaire, en effectuant une rotation dont le centre est le centre du segment joignant le bord antéro-inférieur et le bord antéro-supérieur du corps vertébral de la vertèbre considérée, la rotation concernant tous les points dont l'ordonnée est supérieure à celle du centre de rotation ;
* et à l'aide d'un cinquième outil, on modifie successivement la hauteur de chaque disque, définie comme la longueur du segment perpendiculaire au plateau supérieur du corps vertébral de la vertèbre sous-jacente, joignant le milieu de ce dernier au point d'intersection avec le plateau inférieur de la vertèbre sus-jacente, en effectuant une translation de tous les points sus-jacents au disque, y compris les points formant le plateau inférieur de la vertèbre, sur une distance définie par l'utilisateur ;
- on calcule un premier nuage de points définissant une courbe passant par les centres des corps vertébraux sur les segments du rachis qui ont été modifiés par la modélisation par rapport à la modélisation du rachis pathologique ;
- on en déduit deux autres nuages de points situés chacun de part et d'autre du premier nuage de points, espacés dans le plan frontal par une distance équivalente à la largeur de la vertèbre multipliée par un facteur X, et situés dans le plan sagittal postérieurement au premier nuage de points à une distance équivalente à la profondeur de la vertèbre dans le plan sagittal multipliée par un facteur Y, chacun desdits autres nuages de points définissant une courbe correspondant à la forme qu'il faut imposer à l'une des tiges dudit couple de tiges d'union, lesdits facteurs X et Y permettant de positionner les courbes de paramétrage des tiges d'union (55, 56) de part et d'autre de la courbe passant par le centre des corps vertébraux dans les plans frontal et sagittal en fonction du type d'instrumentation (57, 58) destinée à être utilisée pour relier la tige correspondante au rachis.

2. Procédé selon la revendication 1, **caractérisé en ce que** lors de l'utilisation du cinquième outil, on combine ladite translation avec une rotation autour du centre de rotation du troisième outil là où il se trouve après la translation effectuée par ledit cinquième outil, pour restituer à la hauteur du disque concerné une hauteur correspondant à celle d'un disque sain, non comprimé.

3. Procédé selon l'une des revendications 1 ou 2, **caractérisé en ce que** ladite modélisation de l'ensemble bassin-rachis est réalisée par des moyens informatiques.

4. Procédé selon l'une des revendications 1 à 3, **caractérisé en ce qu'**on convertit lesdits deux autres nuages de points en données exploitables par un dispositif de cintrage d'une tige tel qu'une machine de cintrage à commande numérique.

5. Procédé selon l'une des revendications 1 à 4, **caractérisé en ce que** ladite catégorie préétablie de dos sains est la classification de Roussouly.

6. Procédé de fabrication d'une tige d'union destinée à être implantée sur le rachis d'un patient souffrant d'une pathologie du rachis au cours d'une arthrodèse rachidienne par voie postérieure, **caractérisé en ce que** :
on exécute le procédé de conception d'un couple de tiges d'union (55, 56) destiné à être implanté sur le rachis d'un patient souffrant d'une pathologie du rachis selon l'une des revendications 1 à 5, pour déterminer la forme que doit prendre ladite tige (55, 56), à partir de l'un desdits deux autres nuages de points ;
et on conforme au moins une tige (55, 56) dudit couple de tiges à l'aide d'une machine de cintrage, pour donner à ladite tige (55, 56) la forme qu'elle doit prendre selon le procédé de la revendication 4.

7. Procédé selon la revendication 6, **caractérisé en ce que** ladite machine de cintrage est une machine à commande numérique à laquelle on a fourni lesdites données qu'elle peut exploiter, obtenues d'après le nuage de points correspondant à ladite tige du couple de tiges (55, 56).

## Patentansprüche

1. Verfahren zum Entwerfen eines Paares von Verbindungsstäben (55, 56), die dazu bestimmt sind, an der Wirbelsäule eines Patienten, der an einer Erkrankung der Wirbelsäule leidet, während einer Wirbelsäulenarthrodese durch posteriores Vorgehen implantiert zu werden, **dadurch gekennzeichnet, dass**:
- ein dreidimensionales Modell der pathologischen Wirbelsäule konstruiert wird, indem wie folgt vorgegangen wird:
* Anfertigen mindestens eines Bildes der Wirbelsäule des zu behandelnden Patienten mittels eines medizinischen Bildgebungsverfahrens, nämlich eines Bildes, das die Wirbelsäule in der Sagittalebene zeigt, und, falls die Erkrankung dreidimensional ist, eines weiteren Bildes, das die Wirbelsäule in der Frontalebene in einer Vorderansicht zeigt, wobei sich der Patient in der gleichen Position wie für die Anfertigung des Bildes in der Sagittalebene befindet, wobei die Bilder mindestens den Bereich der Wirbelsäule abdecken, der sich vom Halswirbel C7 bis zum Ende des Kreuzbeins erstreckt und die Femurköpfe des Patienten einschließen;
* Ermitteln morphologischer Bezugspunkte aus dem oder den Bildern, indem Punkte auf den vier Spitzen jedes in zweidimensionaler Projektion in der Aufnahmeebene jedes Bildes betrachteten Wirbelkörpers so angeordnet werden, dass die Konturen der Wirbelkörper auf jedem Bild durch ein Viereck definiert werden;
* Erstellen, anhand des Bildes oder der Bilder, eines vereinfachten dreidimensionalen Modells jedes Wirbelkörpers in Form eines elliptischen Kegelstumpfs, wobei jeder Wirbelkörper in der Ebene des Bildes oder in den jeweiligen Ebenen der Bilder durch ein Viereck dargestellt wird, das durch vier Punkte (1, 2, 3, 4; 5, 6, 7, 8) definiert ist, die auf den vier Spitzen jedes in zweidimensionaler Projektion in der Ebene oder den Ebenen betrachteten Wirbelkörpers angeordnet sind, wobei jede Wirbelplatte durch eine Ellipse dargestellt wird;
* Definieren des Sakralplateaus (15) durch ein Segment in der Sagittalebene, wenn es keine Verformung der Wirbelsäule in der Frontalebene gibt, oder durch eine elliptische Scheibe in der Sagittalebene, wenn es eine Verformung der Wirbelsäule in der Frontalebene auf seinem Niveau gibt, und durch eine elliptische Scheibe in der Frontalebene in allen Fällen, um die Neigung des Kreuzbeins in der Sagittalebene zu berücksichtigen;
* Definieren der Projektionen der Konturen der Femurköpfe in den Aufnahmeebenen der Bilder durch Kreise (9, 10);
* Definieren des Segments, das die Mitte der Kreise (9, 10), die die Femurköpfe darstellen, verbindet, wenn ihre Projektionen in der Sagittalebene versetzt sind, oder den Punkt, der die Mitte der Kreise darstellt, wenn sie in der Sagittalebene zusammenfallen;
* Messen der Beckeninzidenz (PI), der Sakralneigung (SS) und der Beckenversion (PT) auf den Bildern;
* Definieren der "C7-Plumbline" (18) der Wirbelsäule auf den Bildern, die als vertikale Bezugsachse herangezogen wird und die durch einen Punkt in der Mitte des Wirbelkörpers von C7 verläuft;
* Definieren des allgemeinen Gleichgewichtsverhältnisses (d/D), wobei d der Abstand zwischen der vertikalen Achse durch die Mitte des Wirbels C7 und der vertikalen Achse durch das posteriore Ende des Segments ist, das die Sakralplatte (15) in der Sagittalebene darstellt, und D der Abstand zwischen einer vertikalen Achse durch das posteriore Ende des Segments ist, das die Sakralplatte (15) in der Sagittalebene darstellt, und die Mitte des Segments, das die Mitten der die Femurköpfe darstellenden Kreise verbindet, oder den Punkt, der die Mitte dieser Kreise darstellt, wenn sie in der Sagittalebene zusammenfallen;
* Definieren der Grenzen der Lordose- und Kyphosekrümmung auf den Bildern und Berechnen der Anzahl der Wirbel, die in der Lumballordose inbegriffen sind;
- das dreidimensionale Modell verwendet wird, um die pathologische Wirbelsäule des Patienten in eine bestimmte Kategorie einer zuvor festgelegten Klassifikation pathologischer oder gesunder Rückentypen einzuordnen, und daraus eine zukünftige korrigierte Position der Wirbel der Wirbelsäule, die man im Bedarfsfall anstrebt zu erreichen, abzuleiten, wobei die zukünftige korrigierte Position in eine zuvor festgelegte Kategorie gesunder Rückentypen fällt;
- die Wirbelsäule des Patienten in ihrer zukünftigen korrigierten Position modelliert wird, wobei die Konfigurationen und Positionen der Wirbelkörper der Wirbelsäule, der Sakralplatte (15) und der Femurköpfe berücksichtigt werden, wobei ausgehend von dem Modell nacheinander fünf Werkzeuge verwendet werden:
* Neuausrichten der Wirbel in der Frontalebene mit Hilfe eines ersten Werkzeugs gemäß einem Prozentsatz der Neuausrichtung, der für jeden Wirbel von 0 %, was keiner Neuausrichtung entspricht, bis zu 100 %, was einer perfekten Parallelisierung der unteren Platten jedes Wirbelkörpers in der Frontalebene und einer vertikalen Ausrichtung der Mitten der unteren Platten entspricht, reicht;
* Durchführen einer Rotation des Modells des Sakralplateaus in der Sagittalebene relativ zur Mitte des Segments, das die Mitten der beiden Kugeln verbindet, die die Femurköpfe symbolisieren, mit einem zweiten Werkzeug, durch Einwirken auf die Korrektur der Version (PT);
* Ändern der Angulation in der Sagittalebene zwischen den benachbarten Platten jeder Bandscheibe, die operiert werden soll, mit Hilfe eines dritten Werkzeugs, durch Durchführen einer Rotation bei jedem Wirbel, wobei das Zentrum der Rotation die Mitte des Segments ist, das den posterior-inferioren Rand der Platte des Wirbelkörpers des darüber liegenden Wirbels und den posterior-superioren Rand des Wirbelkörpers des darunter liegenden Wirbels verbindet, und wobei die Rotation alle Punkte oberhalb der betreffenden Bandscheibe, einschließlich der beiden Punkte der unteren Platte des Wirbelkörpers des darüber liegenden Wirbels, betrifft;
* Ändern der Angulation zwischen der oberen und unteren Platte von ein bis zwei Wirbeln in der Sagittalebene mit einem vierten Werkzeug, wie dies bei einer transpedikulären Osteotomiekorrektur der Fall wäre, durch Durchführen einer Rotation, deren Zentrum die Mitte des Segments ist, das den anterior-inferioren Rand und den anterior-superioren Rand des Wirbelkörpers des entsprechenden Wirbels verbindet, wobei die Rotation alle Punkte betrifft, deren Ordinate größer als die des Rotationszentrums ist;
* und schrittweises Ändern der Höhe jeder Bandscheibe mit Hilfe eines fünften Werkzeugs, die als die Länge des Segments, das senkrecht zur oberen Platte des Wirbelkörpers des darunter liegenden Wirbels verläuft und die Mitte desselben am Schnittpunkt mit der unteren Platte des darüber liegenden Wirbels verbindet, durch Translation aller oberhalb der Bandscheibe liegenden Punkte, einschließlich der Punkte, die die untere Platte des Wirbels bilden, um eine vom Benutzer definierte Distanz;
- eine erste Punktwolke berechnet wird, die eine Kurve definiert, die durch die Mitten der Wirbelkörper auf den Segmenten der Wirbelsäule verläuft, die durch das Modell relativ zum Modell der pathologischen Wirbelsäule verändert wurden;
- daraus zwei weitere Punktwolken abgeleitet werden, die sich jeweils auf beiden Seiten der ersten Punktwolke befinden, die in der Frontalebene in einem Abstand entfernt sind, der der Breite des Wirbels, multipliziert mit einem Faktor X, entspricht, und sich in der Sagittalebene hinter der ersten Punktwolke in einem Abstand befinden, der der Tiefe des Wirbels in der Sagittalebene, multipliziert mit einem Faktor Y, entspricht, wobei jede der anderen Punktwolken eine Kurve definieren, die der Form entspricht, die einer der Stäbe des Paares der Verbindungsstäbe annehmen muss, wobei die Faktoren X und Y erlauben, die Parametrierkurven der Verbindungsstäbe (55, 56) auf beiden Seiten der Kurve, die durch die Mitte der Wirbelkörper in der Frontal- und Sagittalebene verläuft, in Abhängigkeit von der Art der Instrumente (57, 58), die zur Verbindung des entsprechenden Stabes mit der Wirbelsäule verwendet werden sollen, zu positionieren.

2. Verfahren nach Anspruch 1, **dadurch gekennzeichnet, dass** bei der Verwendung des fünften Werkzeugs die Translation mit einer Rotation um das Rotationszentrum des dritten Werkzeugs dort kombiniert wird, wo es sich nach der durch das fünfte Werkzeug durchgeführten Translation befindet, um in der Höhe der betroffenen Bandscheibe eine Höhe zu rekonstruieren, die der einer gesunden, nicht komprimierten Bandscheibe entspricht.

3. Verfahren nach einem der Ansprüche 1 oder 2, **dadurch gekennzeichnet, dass** die Modellierung der Becken-Wirbelsäulen-Einheit durch Rechnermittel erfolgt.

4. Verfahren nach einem der Ansprüche 1 bis 3, **dadurch gekennzeichnet, dass** die beiden anderen Punktwolken in Daten umgewandelt werden, die von einer Vorrichtung zum Biegen eines Stabs, wie einer numerisch gesteuerten Biegemaschine, verwendet werden können.

5. Verfahren nach einem der Ansprüche 1 bis 4, **dadurch gekennzeichnet, dass** die voreingestellte Kategorie gesunder Rücken die Klassifikation nach Roussouly ist.

6. Verfahren zur Herstellung eines Verbindungsstabs, der dazu bestimmt sind, an der Wirbelsäule eines Patienten, der an einer Erkrankung der Wirbelsäule leidet, während einer Wirbelsäulenarthrodese durch posteriores Vorgehen implantiert zu werden, **dadurch gekennzeichnet, dass**:
das Verfahren zum Entwerfen eines Paares von Verbindungsstäben (55, 56), die dazu bestimmt sind, an der Wirbelsäule eines Patienten, der an einer Erkrankung der Wirbelsäule leidet, nach einem der Ansprüche 1 bis 5 durchgeführt wird, um die Form, die der Stab (55, 56) annehmen muss, aus einer der beiden anderen Punktwolken zu bestimmen;
und mindestens ein Stab (55, 56) des Stabpaares mit Hilfe einer Biegemaschine geformt wird, um dem Stab (55, 56) die Form zu geben, die er gemäß dem Verfahren nach Anspruch 4 annehmen muss.

7. Verfahren nach Anspruch 6, **dadurch gekennzeichnet, dass** die Biegemaschine eine numerisch gesteuerte Maschine ist, der die Daten bereitgestellt werden, die sie verwenden kann und die aus der Punktwolke gewonnenen wurden, die dem Stab des Stabpaares (55, 56) entspricht.

## Claims

1. A method of designing a pair of fixation rods (55, 56) for implantation in the spine of a patient suffering from spinal pathology during posterior spinal arthrodesis, **characterised in that**:
- a three-dimensional model of the pathological spine is constructed as follows:
* at least one image of the patient's spine to be treated is taken by means of a medical imaging method, namely an image showing the spine in the sagittal plane and, if said pathology is three-dimensional, another image showing the spine in the frontal plane in anterior view, the patient being in the same position as for image in the sagittal plane, said images covering at least the area of the spine extending from the cervical vertebra C7 to the end of the sacrum and including the femoral heads of the patient;
* from said image(s), morphological reference points are acquired by placing points on the four vertices of each vertebral body seen in two-dimensional projection in the plane in which each image was taken, so as to define the contours of said vertebral bodies on each image by a quadrilateral;
* a simplified three-dimensional model of each vertebral body is constructed from said image(s) in the form of an elliptical cone frustum for which each vertebral body is represented in the plane of said image(s), or in the respective planes of said image, by a quadrilateral defined by four points (1, 2, 3, 4 ; 5, 6, 7, 8) placed on the four vertices of each vertebral body seen in two-dimensional projection in said plane(s), where each vertebral body is represented by an ellipse;
* the sacral plateau (15) is defined by a segment in the sagittal plane, if there is no deformation of the spine in the frontal plane, or by an elliptical disc in the sagittal plane if there is a deformation of the spine in the frontal plane at its level, and by an elliptical disc in the frontal plane in all cases to take account of the inclination of the sacrum in the sagittal plane;
* the projections of the contours of the femoral heads in the planes of the images are defined by circles (9, 10);
* the segment that connects the centre of the circles (9, 10) representing the femoral heads if their projections in the sagittal plane are offset, or the point representing the centre of said circles if they are merged in the sagittal plane, is defined;
* pelvic incidence (PI), sacral slope (SS) and pelvic tilt (PT) are measured in the images;
* the "C7 plumbline" (18) of the spine is defined on the images and taken as the vertical axis of reference, passing through a point in the middle of the C7 vertebral body;
* the general balance ratio (d/D) is defined, where d is the distance between the vertical axis passing through the middle of the C7 vertebra and the vertical axis passing through the posterior end of the segment representing the sacral plateau (15) in the sagittal plane, and D is the distance between a vertical axis passing through said posterior end of the segment representing the sacral plateau in the sagittal plane and the middle of the segment which connects the centres of the circles representing the femoral heads or the point representing the centre of said circles if they are merged in the sagittal plane;
* the limits of the lordotic and kyphotic curvatures are defined on the images and the number of vertebrae included in the lumbar lordosis is calculated.
- said three-dimensional model is used to classify the patient's pathological spine into a given category of a pre-established classification of pathological or healthy back types, and a future corrected position of the vertebrae of the spine is derived therefrom and aimed at, if necessary, said future corrected position falling within a pre-established category of healthy back types;
- the patient's spine is modelled in its future corrected position, taking into account the configurations and positions of the vertebral bodies of the spine, the sacral plateau (15) and the femoral heads, using five tools in succession on the basis of said three-dimensional model:
* using a first tool, the vertebrae are realigned in the frontal plane, according to a percentage of realignment ranging, for each vertebra, from 0%, which corresponds to an absence of realignment, to 100%, which corresponds to perfect parallelism of the lower plates of each vertebral body in the frontal plane and to vertical alignment of the middles of said lower plates;
* using a second tool, the model of the sacral plateau is rotated in the sagittal plane with respect to the centre of the segment joining the centres of the two spheres symbolising the femoral heads by altering the correction of the pelvic tilt (PT);
* using a third tool, the angulation in the sagittal plane between the adjacent plates of each intervertebral disc to be operated on is modified by performing a rotation on each vertebra, the centre of the rotation being the centre of the segment joining the posteroinferior edge of the vertebral body plate of the overlying vertebra and the posterosuperior edge of the vertebral body plate of the underlying vertebra, and the rotation concerning all points overlying the intervertebral disc under consideration, including the two points of the lower vertebral body plate of the overlying vertebra;
* using a fourth tool, the angulation between the upper and lower plates of one or two vertebrae is modified in the sagittal plane, as would be done during a transpedicular osteotomy correction, by performing a rotation whose centre is the centre of the segment joining the anteroinferior edge and the anterosuperior edge of the vertebral body of the vertebra in question, the rotation concerning all the points whose ordinate is greater than that of the centre of rotation;
* and using a fifth tool, the height of each disc, defined as the length of the segment perpendicular to the upper plate of the vertebral body of the underlying vertebra, joining the middle of the latter to the point of intersection with the lower plate of the overlying vertebra, is modified in succession by performing a translation for all the points overlying the disc, including the points forming the lower plate of the overlying vertebra, over a user-defined distance;
- a first point cloud is calculated defining a curve passing through the centres of the vertebral bodies on the segments of the spine that have been modified by the model compared to the model of the pathological spine;
- two other point clouds are deduced, each located on either side of the first point cloud, spaced in the frontal plane by a distance equivalent to the width of the vertebra multiplied by a factor X, and located in the sagittal plane posterior to the first point cloud at a distance equivalent to the depth of the vertebra in the sagittal plane multiplied by a factor Y, each of said other point clouds defining a curve corresponding to the shape to be imposed on one of the rods of said pair of fixation rods, said factors X and Y making possible to position the configuration curves of the fixation rods (55, 56) on either side of the curve passing through the centre of the vertebral bodies in the frontal and sagittal planes as a function of the type of instrumentation (57, 58) intended to be used to connect the corresponding fixation rod to the spine.

2. A method according to claim 1, **characterised in that** when using the fifth tool, said translation is combined with a rotation about the centre of rotation of the third tool where it is located after the translation performed by said fifth tool, to restore the height of the disc concerned to a height corresponding to that of a healthy, uncompressed disc.

3. A method according to one of claims 1 or 2, **characterised in that** said three-dimensional model of the pathological spine is carried out by computer means.

4. A method according to any of claims 1 to 3, **characterised in that** said two other point clouds are converted into data usable by a rod bending device such as a numerical control bending machine.

5. A method according to any of claims 1 to 4, **characterised in that** said pre-established category of healthy back types is the Roussouly classification.

6. A method of manufacturing a fixation rod for implantation in the spine of a patient suffering from spinal pathology during posterior spinal arthrodesis, **characterised in that**:
the method of designing a pair of fixation rods (55, 56) to be implanted on the spine of a patient suffering from a spinal pathology according to one of claims 1 to 5, is carried out to determine the shape to be taken by said fixation rod (55, 56), from one of said two other point clouds;
and shaping at least one fixation rod (55, 56) of said pair of fixation rods by means of a bending machine, to give said fixation rod (55, 56) the shape it is to take according to the method of claim 4.

7. A method according to claim 6, **characterised in that** said bending machine is a numerical control machine to which has been supplied said data which it can operate, obtained from the point cloud corresponding to said at least one fixation rod of the pair of fixation rods (55, 56).
